# FASCICULE DE BREVET EUROPEEN

(11) **EP 1 627 631 B1**
(45) Date de publication et mention de la délivrance du brevet: **08.02.2012**
(21) Numéro de dépôt: 05111290.2
(22) Date de dépôt: 08.06.2000
(51) Int. Cl.: A61K 9/50, A61K 31/485

(54) **Microgranules de sulfate de morphine, procédé de préparation et composition les contenant**
Morphinsulfat enthaltende Mikrogranulate, Verfahren zu deren Herstellung und pharmazeutische Zusammensetzungen
Morphine sulphate microgranules, method for preparing same and composition comprising same

(30) Priorité: 09.06.1999 FR 9907259
(43) Date de publication de la demande: 22.02.2006
(62) Demande divisionnaire de: 00940460.9
(73) Titulaire: ETHYPHARM, 78550 Houdan (FR)
(72) Inventeur: Marechal, Dominique, Auteuil, Quebec H7H 2W8 (CA); Oury, Pascal, 78150 LE CHESNAY (FR); Suplie, Pascal, 27400 MONTAURE (FR)
(74) Mandataire: Warcoin, Jacques

(56) Documents cités:
- EP-A- 0 377 518
- EP-A- 0 647 448
- FR-A- 2 771 291

## Description

La présente invention concerne une nouvelle formulation du sulfate de morphine à libération prolongée pour administration orale.

La présente invention s'étend en outre au procédé de fabrication de cette formulation et aux préparations pharmaceutiques la contenant.

Dans la présente demande, on entend par "sulfate de morphine" le sel de sulfate, éventuellement hydraté, du (5 alpha, 6 alpha)-7,8-didéhydro-4,5-époxy-17-méthylmorphinan-3,6-diol.

L'administration de sulfate de morphine par voie orale est le traitement le mieux adapté pour soulager des douleurs chroniques. De nombreuses formulations orales de sulfate de morphine ont été décrites dans l'art antérieur.

EP 205 282 (EUROCELTIQUE) porte sur des granules comprenant le sulfate de morphine, un alcool aliphatique et une hydroxyalkylcellulose hydrosoluble.

Ces granules sont enrobés d'un dérivé de cellulose mucoadhésif, comme l'hydroxypropylméthylcellulose et présentent un profil de libération sur 12 heures, avec un pic plasmatique situé entre 1 et 3 heures.

EP 377 518 (FAULDING) décrit des granules à libération prolongée contenant un principe actif très hydrosoluble, comme la morphine. Les granules permettent de maintenir des taux plasmatiques supérieurs à 75 % du maximum pendant au moins 3 heures.

Ces granules comprennent un noyau actif enrobé d'une couche polymérique permettant une libération lente du principe actif à un pH très acide et une libération constante moins lente du principe actif à un pH moins acide à basique sur une période de temps étendue.

Cette couche polymérique contient trois composés : une matrice polymérique insoluble quelque soit le pH, un polymère entérique dont la solubilité est pH dépendante et un polymère soluble en milieu acide.

Les préparations décrites dans EP 377 518 présentent une biodisponibilité nécessitant une administration qui doit être au moins bi-journalière.

EP 553 392 (EUROCELTIQUE) a pour objet un procédé de préparation d'une formulation stable à libération prolongée constituée des granules obtenus en lit d'air fluidisé par pulvérisation d'une solution aqueuse de principe actif sur des neutres, suivi d'un enrobage par l'HPMC, d'un enrobage avec un polymère acrylique et d'un film de protection nécessaire pour réduire l'agglomération des granules.

EP 636 366 (EUROCELTIQUE) décrit des microgranules de sulfate de morphine à libération prolongée comprenant un noyau neutre enrobé d'une couche active constituée d'un mélange principe actif / HPMC, d'une couche à libération prolongée constituée d'Eudragit^{®} RS D et/ou d'Eudragit^{®} RL D, et d'un film d'HPMC, qui représente 5 % de gain de masse.

Dans les documents EP 533 392 et EP 636 366 les granules subissent un traitement thermique au dessus de la température de transition vitreuse de l'enrobage polymérique afin de stabiliser sa structure. Ce traitement thermique est effectué à 45°C environ pendant au moins 24 heures, ce qui allonge considérablement la durée du procédé.

EP 647 448 (EUROCELTIQUE) décrit des granules de sulfate de morphine dont le profil de dissolution in vitro s'étend sur 24 heures. Les granules sont constitués de neutres enrobées de principe actif et de lactose. La couche active est recouverte d'un film d'Opadry^{®} puis enrobée avec de l'Aquacoat ECD 30^{®}, de l'Eudragit RS 30 D^{®} ou un mélange Eudragit RS^{®}/Eudragit RL^{®} : 97,5 / 2,5. Le titre des granules décrits dans ce document est assez faible, de l'ordre de 15 %.

US 5 445 829 (KV Pharmaceutical) porte sur une formulation capable de relarguer le principe actif exclusivement entre 12 et 24 heures après l'administration.

Cette formulation contient 0 à 50 % de particules immédiates et le complément de particules à libération contrôlée, constituées de particules immédiates enrobées d'un dérivé de cellulose comme polymère retardateur.

WO 94/22431 (KAPIPHARMACIA) décrit une formulation à libération contrôlée d'un sel de morphine.

Cette formulation peut être administrée en une seule prise journalière. A 32 heures, la concentration plasmique est supérieure à Cmax/2 et les fluctuations du profil de libération sont très faibles sur cette période si bien que la concentration plasmatique est quasiment constante sur 24 heures.

La formulation décrite dans WO 94/22431 est par exemple constituée de granules contenant un coeur de sel de morphine, de lactose et d'un liant, enrobé d'un film d'HPMC/EC et de triéthylcitrate.

Cette formulation utilise un mélange de deux polymères, l'un étant soluble et l'autre insoluble dans l'eau.

WO 95/31972 (EUROCELTIQUE) décrit des granules de sulfate morphine à libération prolongée constitués d'un noyau neutre enrobé de principe actif et de lactose hydraté dont la densité apparente est comprise entre 0,4 et 0,9 g/ml. La couche à libération retardée enrobant le principe actif contient par exemple un polymère acrylique, une alkylcellulose, une huile végétale hydrogénée ou un de leurs mélanges.

Ce document enseigne que la fixation du sulfate de morphine sur les noyaux neutres nécessite l'adjonction du lactose comme diluant.

Les profils de libération des microgranules donnés en exemple montrent que ces granules sont adaptés à une prise jour.

WO 96/14059 (EUROCELTIQUE) décrit un procédé d'extrusion de particules sphériques contenant du sulfate de morphine, un support dont le point de fusion est compris entre 35 et 150°C, un agent à libération prolongée.

Le support est une huile végétale hydrogénée ou un PEG (Mw 1000 - 20000). Le profil de dissolution in vitro de ces particules est de 67 % à 24 heures. Aucun résultat in vitro n'est fourni.

WO/960066 (ALZA) décrit une composition contenant du sulfate de morphine, de la polyvinylpyrrolidone et un polyoxyde d'alkylène.

Ce document prétend que la formulation procure une libération prolongée dans le temps mais ne donne aucun exemple ni in vitro ni in vivo, si bien qu'il est difficile à la lecture du document d'estimer si l'administration doit être d'une ou de plusieurs prises jour.

L'invention a pour objet des microgranules de sulfate de morphine à libération prolongée comprenant chacun un grain support neutre enrobé d'une couche active et d'une couche à libération prolongée, caractérisés en ce que leur composition est la suivante :
- Grain de Support neutre 30 - 40 %
- Couche active
   - Sulfate de morphine 30 - 40 %
   - Liant 10 - 20 %
- Couche à libération prolongée
   - Copolymère d'acide méthacrylique 5 - 15 %
   - Plastifiant 1- 2,5 %
   - Lubrifiant 2 - 4 %
   - Silice hydrophobe 0,2 -1 %

On préfère l'Aerosil^{®} R 972 comme silice hydrophobe.

Les microgranules de l'invention présentent notamment l'avantage d'être dépourvus d'un film de protection enrobant la couche à libération prolongée. En outre, il n'est pas nécessaire de faire subir aux microgranules un traitement thermique de très longue durée (supérieure à 24 heures) comme dans l'art antérieur pour améliorer la structure de la couche à libération prolongée.

La proportion massique relative du sulfate de morphine et du grain support neutre est de préférence comprise entre 40/60 et 60/40.

et un lubrifiant. Le plastifiant et le lubrifiant sont choisis parmi les plastifiants et les lubrifiants pharmaceutiquement acceptables bien connus de l'homme du métier. Le plastifiant est par exemple le triéthylcitrate.

Les grains supports neutres sont une granulométrie comprise entre 200 et 1000 µm, de préférence entre 400 et 600 µm.

La présente invention concerne également un procédé de préparation des microgranules décrits précédemment. Ce procédé est entièrement réalisé en milieu aqueux. Il comprend une étape de montage en solution aqueuse du principe actif sur des grains supports neutres, et une étape d'enrobage avec un copolymère méthacrylique, toujours en solution aqueuse.

Les granules sont avantageusement préparés dans une turbine rotative perforée ou un lit d'air fluidisé. La pulvérisation des solutions et/ou suspensions de montage et d'enrobage est de préférence continue et suivie d'une étape de séchage à une température comprise entre 30 et 65°C.

Il n'est pas nécessaire que les granules selon l'invention subissent un traitement thermique pour que la structure du film soit satisfaisante.

La présente invention concerne enfin les compositions pharmaceutiques contenant les microgranules de l'invention éventuellement obtenus selon le procédé décrit précédemment.

Les exemples suivants illustrent l'invention sans en limiter la portée.

Les pourcentages sont exprimés en poids.

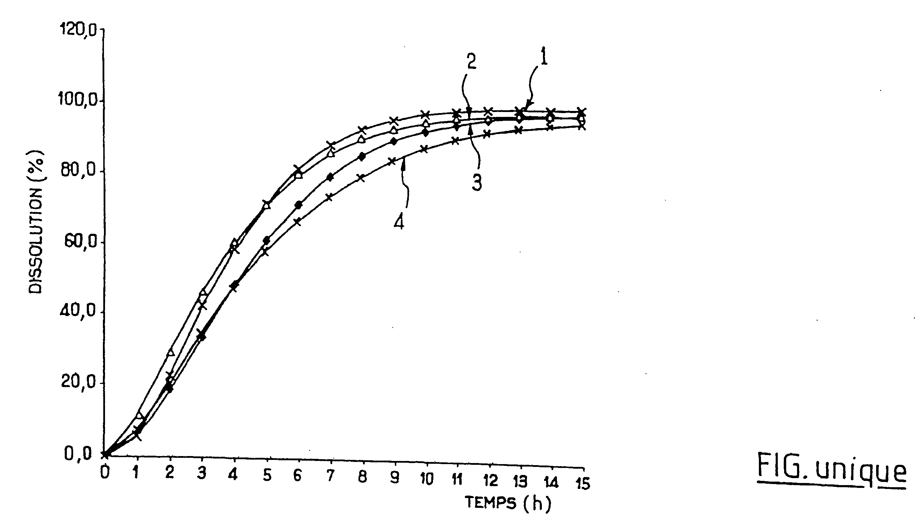

La figure représente la moyenne du profil de dissolution in vitro de quatre formulations selon l'invention (courbes 1, 2, 3 et 4). Le pourcentage de dissolution est en abscisse et le temps (heures) en ordonnée.

### Exemple 1 fLot A)

### • Préparation des granules

On prépare une solution de montage contenant 74,7 % d'eau purifiée, 6,6% de Pharmacoat 603^{®} (hydroxypropylméthylcellulose) et 18,7 % de sulfate de morphine. L'agitation est maintenue jusqu'à homogénéité de la solution puis pendant tout le montage.

Des grains supports neutres (400-600 µm) sont placés dans une turbine perforée en rotation. Le montage du principe actif est effectué sur les neutres par pulvérisation continue de la solution de montage décrite précédemment, avec un support d'air chaud à une température comprise entre 35 et 60°C.

La masse des microgranules actifs obtenue est tamisée sur une grille d'ouverture de maille allant de 0,71 à 0,85 mm.

On prépare une suspension d'enrobage en ajoutant successivement dans de l'eau purifiée, de l'Eudragit^{®} RS 30 D (copolymère d'acide méthacrylique), du triéthylcitrate, du talc et de l'Aerosil^{®} R 972 (silice hydrophobe). L'agitation de la suspension est maintenue jusqu'à homogénéité du mélange, puis durant tout l'enrobage.

Les microgranules actifs sont placés dans une turbine perforée en rotation et pulvérisés de façon continue avec la suspension d'enrobage décrite précédemment, à une température de 30°C. La masse de microgranules obtenus est tamisée sur une grille d'ouverture de maille allant de 0,8 à 1 mm.

Cette étape peut être répétée une ou plusieurs fois. Les granules sont ensuite lubrifiés avec une quantité de talc équivalente à 0,5 % de la masse enrobée obtenue.

Les microgranules obtenus ont la composition suivante :

| | **Lot A** | |
|---|---|---|
| | **Quantité mg** | **% massique** |
| Sulfate de morphine | 12,5 | 37,3 |
| Neutres | 12,5 | 37,3 |
| Pharmacoat 603^{®} | 4,4 | 13,0 |
| Eudragit RS 30 D^{®} | 2,7 | 8,2 |
| Triéthyl citrate | 0,5 | 1,6 |
| Talc | 0,7 | 2,1 |
| Aerosil R 972^{®} | 0,1 | 0,4 |
| Teneur (mg/g) | 371 | |

### • Essais de dissolution in vitro

Les microgranules obtenus précédemment sont dissous dans 500 ml d'eau à 37°C dans un appareil à palettes tournant à 100 tours/min. La lecture de l'absorbance U.V. est mesurée à deux longueurs d'onde 285 nm et 310 nm.

| | **Lot A** | | | | | | | | | | | |
|---|---|---|---|---|---|---|---|---|---|---|---|---|
| **Temps (heures)** | **1** | **2** | **3** | **4** | **5** | **6** | **7** | **8** | **9** | **10** | **15** | **20** |
| **Pourcentage de dissolution** | 6,6 | 20,8 | 38,8 | 55,8 | 69,9 | 79,9 | 86,3 | 90,7 | 93,2 | 94,8 | 97,8 | 98,3 |

Le profil de dissolution in vitro du lot A est représenté par la courbe 3 de la figure.

### • Essais de stabilité des gélules de microgranules (lot A1)

Les propriétés de stabilité des microgranules obtenus précédemment et conditionnés en gélules de taille 3 contenant 60 mg de sulfate de morphine chacune, sont mesurées dans des conditions de stockage à 25°C et 60% d'humidité relative, pendant 24 mois.

On observe que la teneur en eau des microgranules est stable à 6 % en moyenne, que l'aspect des gélules est satisfaisant, et que le titre en principe actif est conforme et homogène.

Les profils de dissolution sont assez stables au cours du temps.

Au bout de 24 mois, la teneur en impuretés pseudomorphine et ampomorphine est conforme aux normes (soit inférieure à 0,5 %).

La stabilité des mêmes gélules est également étudiée pendant 6 mois à 40°C et 75 % d'humidité relative.

On observe que le titre en principe actif est conforme et homogène. La dissolution est stable à 6 mois. Par ailleurs, la teneur en eau est stable.

Les résultats de stabilité sont présentés dans les tableaux suivants.

| **Pourcentage de dissolution in vitro (lot A1)** Conditions de stockage 25°C, 60 % HR | | | | | | | | |
|---|---|---|---|---|---|---|---|---|
| **Heures** | **T0** | **1M** | **3M** | **6M** | **9M** | **12M** | **18M** | **24M** |
| 1 | 7,8 | 7,4 | 7,7 | 7,1 | 6,1 | 6,5 | 6,4 | 5,5 |
| 2 | 21,6 | 21,9 | 23,2 | 22,4 | 18,9 | 19,7 | 20,1 | 17,0 |
| 4 | 55,2 | 57,3 | 60,2 | 58,1 | 52,7 | 53,1 | 52,9 | 50,6 |
| 6 | 78,9 | 81,7 | 83,7 | 81,0 | 77,8 | 76,1 | 73,4 | 76,1 |
| 8 | 89,9 | 93,4 | 93,8 | 90,8 | 90,1 | 86,7 | 81,9 | 88,5 |
| 12 | 96,0 | 100,2 | 98,8 | 95,9 | 97,5 | 93,0 | 86,2 | 95,4 |
| 16 | 96,4 | 100,6 | 99,8 | 96,9 | 98,7 | 94,6 | 86,9 | 95,4 |

| **Pourcentage de dissolution in vitro (lot A1)** Conditions de stockage 40°C, 75 % HR | | | | | |
|---|---|---|---|---|---|
| **Heures** | **T0** | **1M** | **2M** | **3M** | **6M** |
| **1** | 7,8 | 6,0 | 5,9 | 6,1 | 6,3 |
| **2** | 21,6 | 19,8 | 19,7 | 19,7 | 21,0 |
| **4** | 55,2 | 57,1 | 57,3 | 57,0 | 58,7 |
| **6** | 78,9 | 83,1 | 81,8 | 81,9 | 83,2 |
| **8** | 89,9 | 94,3 | 92,1 | 92,9 | 94,0 |
| **12** | 96,0 | 100,1 | 97,5 | 98,7 | 100,3 |
| **16** | 96,4 | 101,5 | 98,0 | 99,6 | 102,4 |

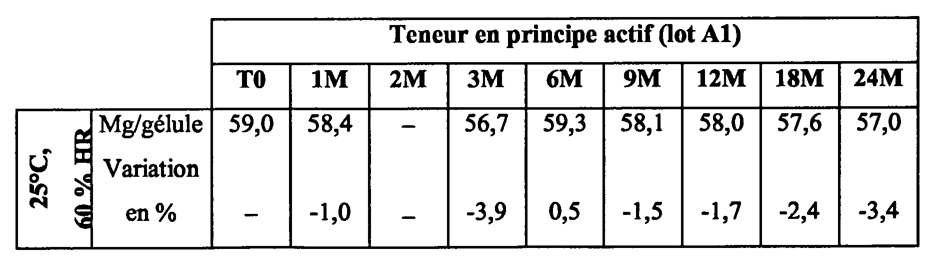

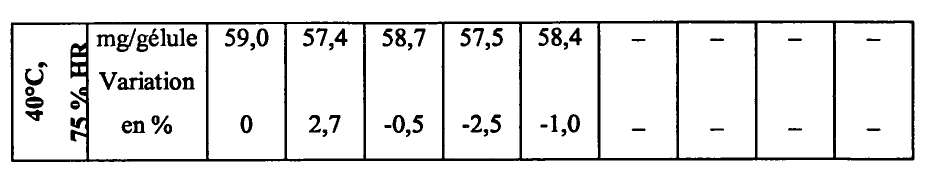

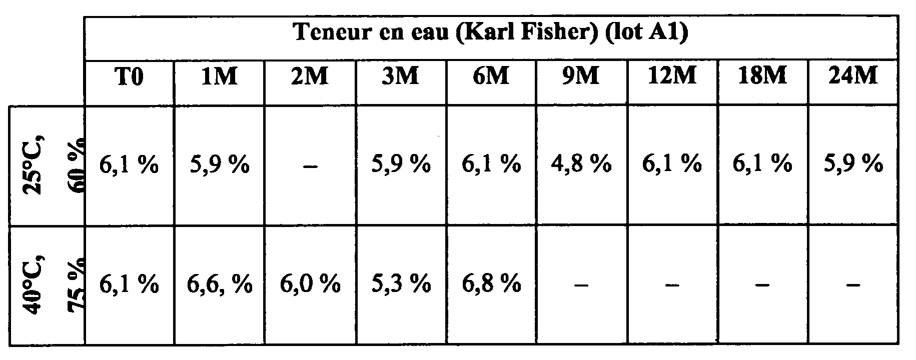

### • Etude pharmacocinétique n°1

On compare la biodisponibilité du lot de gélules A1 à celle d'une formulation de morphine de référence (dosée à 30 mg), après administration d'une dose répétée de 7 jours chez 24 volontaires sains

| | **Concentration plasmatique en** | | | |
|---|---|---|---|---|
| | **Morphine** | | **6-glucuronide-morphine** | |
| | **Gélules de microgranules (lot A1) 60 mg** | **Référence (lot S 1079) 30 mg** | **Gélules de microgranules (lot A1) 60 mg** | **Référence (lot S 1079) 30 mg** |
| Cₘₐₓ (ng/ml)* | 18,3 | 12,8 | 77,6 | 59,2 |
| Cₘᵢₙ (ng/ml)** | 7,9 | 6,8 | 31,0 | 30,4 |
| Tₘₐₓ (h)* | 5 | 5 | 6 | 3 |

| | | | | |
|---|---|---|---|---|
| *moyennes ** médianes | | | | |

On remarque qu'à J7, les concentrations plasmatiques en morphine à 24 heures des gélules de l'invention sont plus élevées que les concentrations plasmatiques de la référence à 12 heures (+ 1,1 ng/ml) ce qui laisse présager une bonne couverture sur 24 heures.

### • Etude pharmacocinétique n°2.

On compare la biodisponibilité de gélules du lot A2 à celle d'une formulation de morphine de référence, après administration d'une dose unique de 60 mg chez des volontaires sains.

Les gélules du lot A2 sont de taille 3 et dosés à 60 mg de sulfate de morphine par gélule.

| | **Concentration plasmatique en** | | | |
|---|---|---|---|---|
| | **Morphine** | | **6-glucuronide-morphine** | |
| | **Gélules de microgranules de l'invention (lot A2)** | **Référence de l'art antérieur (lot S 1055)** | **Gélules de microgranules de l'invention (lot A2)** | **Référence de l'art antérieur (lot S 1055)** |
| Cₘₐₓ (ng/ml)* | 6,97 | 13,16 | 64,0 | 114,8 |
| Cₘᵢₙ (ng/ml)** | 6,0 | 2,0 | 5,0 | 3,0 |
| Tₘₐₓ (h)* | 218,9 | 186,9 | 1471,49 | 1536,5 |

| | | | | |
|---|---|---|---|---|
| *moyennes ** médianes | | | | |

La formulation de l'invention et la référence sont bioéquivalentes sur les paramètres d'aires sous courbes, ce qui démontre une absorption équivalente des deux produits. En revanche, le profil de libération de la formulation de l'invention apparaît plus retard que la référence avec un Tₘₐₓ plus tardif et un Cₘₐₓ plus bas.

### Exemple 2 (lots B, C et D)

### • Préparation des granules

On prépare des granules de composition suivante en suivant le protocole de l'exemple 1.

| | **Lot B** | | **Lot C** | | **Lot D** | |
|---|---|---|---|---|---|---|
| | **Quantité (kg)** | **% massique** | **Quantité (kg)** | **% massique** | **Quantité (g)** | **% massique** |
| Sulfate de morphine | 13,7 | 35,1 | 31,0 | 40,9 | 728,8 | 41,9 |
| Neutres | 15,4 | 39,7 | 26,0 | 34,3 | 573,7 | 33,0 |
| Pharmacoat 603^{®} | 4,8 | 12,3 | 10,8 | 14,3 | 204,1 | 11,7 |
| PEG 4000 | - | - | - | - | 51,0 | 2,9 |
| Eudragit RS 30 D^{®} | 3,2 | 8,2 | 5,1 | 6,7 | 126,5 | 7,3 |
| Triéthyl citrate | 0,6 | 1,6 | 1,0 | 1,3 | 24,9 | 1,4 |
| Talc | 1,0 | 2,6 | 1,7 | 2,2 | 24,9 | 1,4 |
| Aerosil^{®} | 0,1 | 0,40 | 0,2 | 0,3 | 6,2 | 0,4 |
| Teneur (mg/g) | 371,3 | | 368,5 | | 397,9 | |

Le lot B est préparé comme dans l'exemple 1 dans une turbine perforée GLATT, tandis que les lots C et D sont respectivement préparés dans une turbine perforée O'HARA et dans un LAF HUTTLIN.

### • Essais de dissolution in vitro des microgranules

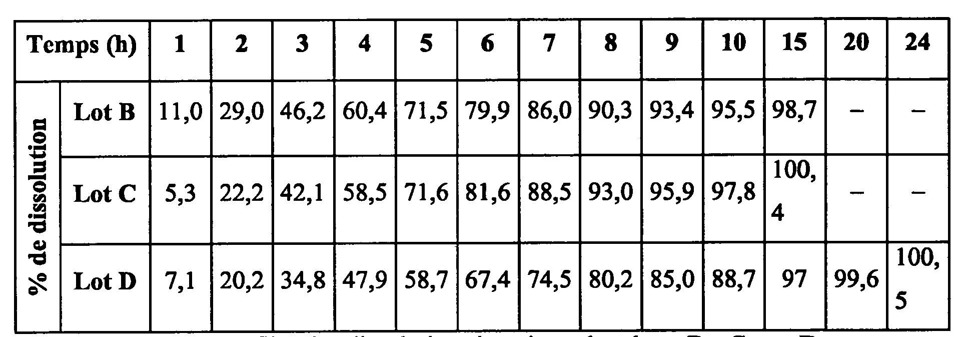

Les profils de dissolution in vitro des lots B, C et D sont respectivement représentés par les courbes 2,1 et 4 de la figure.

### • Essais de dissolution des gélules de microgranules

Les gélules des lots B2, B1, D1 et C1 sont dosées à 60 mg de sulfate de morphine.

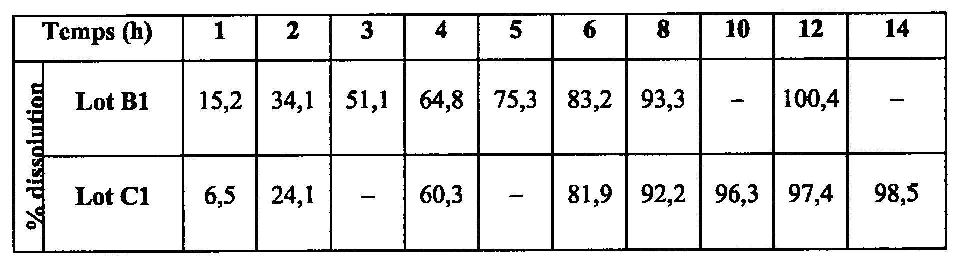

### • Essais de stabilité à 25°C, 60 % HR du lot de gélules B2 (microgranules du lot B)

| | **T0** | **15J** | **1M** | **2M** | **3M** | **6M** |
|---|---|---|---|---|---|---|
| **Teneur en eau (%)** | - | 5,50 % | 6,00 % | 6,16 % | 6,00 % | 6,02 % |

| **Dissolution (heures)** | | | | | | |
|---|---|---|---|---|---|---|
| **1** | 21,2 | 19,2 | 14,7 | 6,9 | 15,6 | 16,6 |
| **2** | 45,1 | 43,1 | 29,5 | 22,1 | 35,7 | 37,9 |
| **3** | 63,5 | 62,0 | 42,9 | 36,7 | 53,3 | 55,8 |
| **4** | 76,1 | 75,7 | 54,4 | 49,4 | 67,1 | 69,3 |
| **5** | 85,2 | 85,2 | 64,0 | 60,1 | 77,3 | 79,3 |
| **6** | 91,3 | 91,6 | 71,9 | 68,8 | 84,8 | 86,5 |
| **7** | 95,5 | 95,7 | 78,2 | 76,0 | 90,3 | 91,5 |
| **8** | 98,2 | 98,4 | 83,6 | 81,5 | 94,1 | 95,0 |
| **12** | 102,2 | 102,9 | 96,3 | 93,1 | 101,2 | 101,0 |

### • Essais de stabilité à 40°C, 75 % HR du lot de gélules D1 (microgranules du lot D)

| | **T0** | **15J** | **1M** | **2M** | **3M** | **6M** |
|---|---|---|---|---|---|---|
| **Teneur en eau (%)** | 6,19 % | 6,40 % | 6,29% | 6,20 % | 6,30 % | 6,38% |

| **Dissolution (heures)** | | | | | | |
|---|---|---|---|---|---|---|
| **1** | 11,8 | 11,9 | 12,2 | 12,6 | 11,6 | 12,5 |
| **2** | 28,7 | 28,7 | 31,0 | 33,1 | 31,6 | 34,3 |
| **3** | 45,8 | 45,2 | 48,1 | 50,6 | 49,1 | 51,8 |
| **4** | 59,3 | 58,4 | 61,2 | 63,9 | 62,5 | 64,9 |
| **5** | 69,8 | 68,8 | 71,5 | 74,1 | 72,8 | 75,2 |
| **6** | 77,9 | 77,1 | 79,6 | 82,1 | 80,7 | 83,0 |
| **8** | 88,5 | 88,8 | 90,3 | 91,9 | 90,8 | 88,7 |
| **10** | 94,2 | 95,5 | 95,4 | 96,0 | 95,0 | 95,7 |
| **12** | 97 | 98,7 | 97,6 | 97,5 | 96,7 | 97,1 |

## Revendications

1. Microgranules de sulfate de morphine à libération prolongée comprenant chacun un grain support neutre enrobé d'une couche active et d'une couche à libération prolongée, **caractérisés en ce que** leur composition est la suivante :
• Grain de Support neutre 30-40 % en poids
• Couche active
- Sulfate de morphine 30-40% en poids
- Liant 10-20% en poids
• Couche à libération prolongée
- Copolymère d'acide méthacrylique 5-15% en poids
- Plastifiant 1-2,5% en poids
- Lubrifiant 2-4% en poids
- Silice hydrophobe 0,2-1% en poids

2. Microgranules selon la revendication 1, **caractérisés en ce que** le copolymère d'acide méthacrylique est le Chlorure de Poly(éthylacrylate, méthyl méthacrylate, triméthylammonioéthyl méthacrylate) 1:2:0,1.

3. Microgranules selon la revendication 1 ou 2, **caractérisés en ce que** le copolymère d'acide méthacrylique est l'Eudragit® RS 30D.

4. Microgranules selon l'une quelconque des revendications 1 à 3, **caractérisés en ce que** la silice hydrophobe est l'Aérosil® R972.

5. Microgranules selon l'une quelconque des revendications 1 à 4, **caractérisés en ce que** le grain support neutre enrobé de la couche active contient 40 % à 50 % en poids de sulfate de morphine et 10 à 20 % en poids d'un liant pharmaceutiquement acceptable.

6. Microgranules selon l'une quelconque des revendications 1 à 5, **caractérisés en ce que** le plastifiant est le triéthylcitrate.

7. Microgranules selon l'une des revendications précédentes, **caractérisés en ce que** la proportion massique relative du sulfate de morphine et du grain support neutre est comprise entre 40/60 et 60/40.

8. Microgranules selon l'une quelconque des revendications précédentes, **caractérisés en ce que** les grains de support neutres ont une granulométrie comprise entre 200 et 1000 µm, de préférence entre 400 et 600 µm

9. Procédé de préparation des microgranules selon l'une des revendications 1 à 8, **caractérisé en ce que** la couche active et la couche à libération prolongée sont appliquées sur les grains neutres, par montage en solution aqueuse.

10. Composition pharmaceutique contenant les microgranules selon l'une des revendications 1 à 8, éventuellement obtenus selon le procédé de la revendication 9.

## Claims

1. Sustained-release morphine sulphate microgranules each comprising a neutral support grain coated with an active layer and a sustained-release layer, **characterized in that** the composition thereof is as follows:
• Neutral support grain 30 - 40% by weight
• Active layer
- Morphine sulphate 30 - 40% by weight
- Binder 10 - 20% by weight
• Sustained-release layer
- Methacrylic acid copolymer 5 - 15% by weight
- Plasticizer 1 - 2.5% by weight
- Lubricant 2 - 4% by weight
- Hydrophobic silica 0.2 - 1% by weight

2. Microgranules according to claim 1, **characterized in that** the methacrylic acid copolymer is 1:2:0.1 Poly(ethylacrylate, methyl methacrylate, trimethylammonioethyl methacrylate) chloride.

3. Microgranules according to claim 1 or 2, **characterized in that** the methacrylic acid copolymer is Eudragit® RS 30D.

4. Microgranules according to any of claims 1 to 3, **characterized in that** the hydrophobic silica is Aerosil® R972.

5. Microgranules according to any of claims 1 to 4, **characterized in that** the neutral support grain coated with the active layer contains 40% to 50% by weight of morphine sulphate and 10 to 20% by weight of a pharmaceutically acceptable binder.

6. Microgranules according to any of claims 1 to 5, **characterized in that** the plasticizer is triethylcitrate.

7. Microgranules according to any of the previous claims, **characterized in that** the relative mass proportion of the morphine sulphate and of the neutral support grain is between 40/60 and 60/40.

8. Microgranules according to any of the previous claims, **characterized in that** the neutral support grains have a grain size distribution between 200 and 1000 µm, preferably between 400 and 600 µm.

9. Process for preparing the microgranules according to any of claims 1 to 8, **characterized in that** the active layer and the sustained-release layer are applied onto the neutral grains by emplacing in aqueous solution.

10. Pharmaceutical composition containing the microgranules according to any of claims 1 to 8 optionally obtained according to the process according to claim 9.

## Patentansprüche

1. Morphinsulfat-Mikrogranulate zur verlängerten Freisetzung, jeweils umfassend ein neutrales Trägerkorn, das von einer aktiven Schicht und einer Schicht zur verlängerten Freisetzung umhüllt ist, **dadurch gekennzeichnet, dass** sie folgende Zusammensetzung aufweisen:
• Neutrales Trägerkorn 30 - 40 Gew.-%
• Aktive Schicht
- Morphinsulfat 30 - 40 Gew.-%
- Bindemittel 10 - 20 Gew.-%
• Schicht zur verlängerten Freisetzung
- Methacrylsäure-Copolymer 5 - 15 Gew.-%
- Weichmacher 1 - 2,5 Gew.-%
- Gleitmittel 2 - 4 Gew.-%
- Hydrophobe Kieselerde 0,2 - 1 Gew.-%

2. Mikrogranulate gemäß Anspruch 1, **dadurch gekennzeichnet, dass** das Methacrylsäure-Copolymer Poly(ethylacrylat-co-methylmethacrylat-co-trimethyl-ammonioethylmethacrylat)-chlorid 1:2:0,1 ist.

3. Mikrogranulate gemäß Anspruch 1 oder 2, **dadurch gekennzeichnet, dass** das Methacrylsäure-Copolymer Eudragit® RS 30 D ist.

4. Mikrogranulate gemäß irgendeinem der Ansprüche 1 bis 3, **dadurch gekennzeichnet, dass** die hydrophobe Kieselerde Aerosil® R972 ist.

5. Mikrogranulate gemäß irgendeinem der Ansprüche 1 bis 4, **dadurch gekennzeichnet, dass** das von der aktiven Schicht umhüllte neutrale Trägerkorn 40 bis 50 Gew.-% Morphinsulfat und 10 bis 20 Gew.-% eines pharmazeutisch akzeptablen Bindemittels enthält.

6. Mikrogranulate gemäß irgendeinem der Ansprüche 1 bis 5, **dadurch gekennzeichnet, dass** der Weichmacher Triethylcitrat ist.

7. Mikrogranulate gemäß irgendeinem der vorhergehenden Ansprüche, **dadurch gekennzeichnet, dass** das Massenverhältnis von Morphinsulfat zu dem neutralen Trägerkorn 40/60 bis 60/40 beträgt.

8. Mikrogranulate gemäß irgendeinem der vorhergehenden Ansprüche, **dadurch gekennzeichnet, dass** die neutralen Trägerkörner eine Körnchengröße von 200 bis 1000 µm, bevorzugt 400 bis 600 µm, aufweisen.

9. Verfahren zur Herstellung von Mikrogranulate gemäß einem der Ansprüche 1 bis 8, **dadurch gekennzeichnet, dass** die aktive Schicht und die Schicht zur verlängerten Freisetzung auf die neutralen Körner durch Zusammenbau in wässriger Lösung aufgebracht werden.

10. Pharmazeutische Zusammensetzung, welche Mikrogranulate gemäß einem der Ansprüche 1 bis 8, die gegebenenfalls durch das Verfahren von Anspruch 9 erhalten wurden, enthält.
